# EUROPÄISCHE PATENTSCHRIFT

(11) **EP 1 270 043 B1**
(45) Veröffentlichungstag und Bekanntmachung des Hinweises auf die Patenterteilung: **02.04.2008**
(21) Anmeldenummer: 02002032.7
(22) Anmeldetag: 07.02.2002
(51) Int. Cl.: A61N 2/02, A61B 5/055

(54) **Verfahren und Vorrichtung für transcraniale magnetische Stimulation und kortikale Kartographie**
Method and device for transcranial magnetic stimulation and cortical cartography
Procédé et dispositif de stimulation magnétique transcranienne et de cartographie corticale

(30) Priorität: 28.06.2001 EP 01114823
(43) Veröffentlichungstag der Anmeldung: 02.01.2003
(73) Patentinhaber: BrainLAB AG, 85551 Kirchheim/Heimstetten (DE)
(72) Erfinder: Tanner, Philipp, 80769 München (DE)
(74) Vertreter: Schwabe - Sandmair - Marx

(56) Entgegenhaltungen:
- ETTINGER G J ET AL: "Non-invasive functional brain mapping using registered transcranial magnetic stimulation" PROCEEDINGS OF THE IEEE WORKSHOP ON MATHEMATICAL METHODS IN BIOMEDICAL IMAGE ANALYSIS (CAT. NO.96TB100056), PROCEEDINGS OF THE WORKSHOP ON MATHEMATICAL METHODS IN BIOMEDICAL IMAGE ANALYSIS, SAN FRANCISCO, CA, USA, 21-22 JUNE 1996, Seiten 32-41, XP002202005 1996, Los Alamitos, CA, USA, IEEE Comput. Soc. Press, USA ISBN: 0-8186-7367-2
- HERWIG U ET AL.: "Neuronavigation in der Phsychatrie - fokussierte transkranielle Magnetstimulation bei depressiven Patienten" NERVENHEILKUNDE, Bd. 18, Nr. 7, Juli 1999 (1999-07), Seiten 353-357, XP008004455
- MARTÍNEZ J M ET AL.: "A fast 3-D model for localizing the precise site of the magnetic stimulation: An application to transcranial magnetic stimulation" 12TH INTERNATIONAL CONFERENCE ON BIOMAGNETISM, HELSINKI UNIVERSITY OF TECHNOLOGY, ESPOO, FINLAND, [Online] 13. - 17. August 2000, XP002201970 Gefunden im Internet: <URL:http://biomag2000.hut.fi/abstracts/tm s.pdf> [gefunden am 2002-06-12]
- RUOHONEN J ET AL: "FOCUSING AND TARGETING OF MAGNETIC BRAIN STIMULATION USING MULTIPLECOILS" MEDICAL AND BIOLOGICAL ENGINEERING AND COMPUTING, PETER PEREGRINUS LTD. STEVENAGE, GB, Bd. 36, Nr. 3, 1. Mai 1998 (1998-05-01), Seiten 297-301, XP000751652 ISSN: 0140-0118

## Beschreibung

Die vorliegende Erfindung bezieht sich auf Verfahren und Vorrichtungen zur transcranialen magnetischen Stimulation (TMS), bevorzugt zur navigierten TMS, insbesondere zur nichtinvasiven Lokalisation und/oder Funktionsbestimmung bestimmter Bereiche eines Gehirns, wie zum Beispiel sogenannter primärer oder sekundärer Hirnareale. Damit können zum Beispiel die Hirnfunktionen kartiert, also bestimmten Hirnbereichen zugeordnet werden, was auch als kortikale Kartographie bezeichnet wird. Ebenso ist es möglich festzustellen, welcher Bereich des Gehirns oder einer Hirnwindung (Gyrus) eine bestimmte Funktion erfüllt.

In verschiedenen medizinischen Bereichen, wie zum Beispiel der Neurologie, der Psychiatrie oder der Gehirn-Chirurgie ist es wünschenswert bestimmte funktionale Bereiche des Gehirns lokalisieren zu können, um Hirnfunktionen zu kartieren. Soll zum Beispiel durch einen chirurgischen Eingriff ein Hirntumor entfernt werden, so sollte der Tumor soweit wie möglich entfernt werden, wobei vor allem sogenannte primäre Hirnareale, welche bezüglich der Motorik, Sensorik, der Sprache oder den visuellen Fähigkeiten einer Person eine entscheidende Rolle spielen, nach Möglichkeit nicht verletzt werden sollen. Diese Bereiche sollten bei einem chirurgischen Eingriff wenn möglich gar nicht oder nur in äußerst geringem Umfang verletzt werden.

Das Auffinden solcher besonderen Hirnbereiche wurde nach einem bekannten direkten Verfahren intra-operativ durchgeführt, wobei mittels Elektroden eine direkte kortikale Stimulation (DCS) an einem geöffneten Schädel erfolgte. Dabei wurde eine Elektrode in einen bestimmten Bereich des Gehirns eingebracht und ein elektrischer Impuls angelegt, wobei die auf den elektrischen Impuls folgende Reaktion der untersuchten Person, zum Beispiel das Zucken eines Muskels oder das Wahrnehmen von visuellen Eindrücken beobachtet wurde. Die durch die direkte kortikale Stimulation aufgefundenen besonderen Hirnbereiche wurden mittels aufgelegter kleiner Plättchen markiert, welche für einen Chirurgen bei einer nachfolgenden Gehimoperation eine Orientierungshilfe bezüglich der möglichst nicht zu verletzenden Himbereiche darstellten. Die direkte kortikale Stimulation stellt bis zum heutigen Tag das präziseste Verfahren zur Kartierung von Hirnfunktionen dar und ermöglicht eine Genauigkeit bei der Auffindung bestimmter Hirnareale im Bereich von wenigen Millimetern. Jedoch kann dieses Verfahren nur intra-operativ durchgeführt werden, wobei die untersuchte Person bei vollem Bewusstsein sein muss. Dies kann bei der Anwendung dieses Verfahrens jedoch zu Problemen führen, da dieser Zustand für die untersuchte Person unangenehm ist und die Person, falls es zu Komplikationen kommen sollte, aufgrund der geöffneten Schädeldecke nicht einfach hingelegt und ruhig gestellt werden kann.

Es sind weiterhin verschiedene indirekte Verfahren zur Kartierung von Hirnfunktionen bekannt, mit welchen jedoch nur eine erheblich geringere Genauigkeit beim Auffinden spezifischer Hirnareale erreicht werden kann. So muss zum Beispiel bei der funktionellen Kernspintomographie (fMRI) eine untersuchte Person bestimmte Aktionen wie zum Beispiel eine Handbewegung ausführen, welche die Durchblutung der diesen Aktionen zugeordneten Hirnbereichen fördert. Diese Veränderung der Durchblutung bestimmter Himbereiche kann aufgrund der Entkopplung von Durchblutung und Sauerstoffverbrauch während der neuronalen Aktivität gemessen werden, da hierdurch eine Hyperoxygenierung und damit ein Abfall der Konzentration des paramagnetischen Deoxy-Hämoglobins (BOLD-Effekt) auftritt, was dann als sogenanntes "endogenes Kontrastmittel" mittels geeigneter Sequenzen mit Kernspintomographie gemessen werden kann. Jedoch ist dieses Verfahren wie oben erwähnt, relativ ungenau und liefert nur eine räumliche Auflösung im Bereich von etwa 0,5 bis 1,0 cm.

Aus Neurosurgery 1992-1998, December 1997, Volume 41, Number 6, 1319 "Stereotactic Transcranial Magnetic Stimulation: Correlation with Direct Electrical Cortical Stimulation" ist ein Verfahren bekannt, bei welchem eine stereotaktische transcraniale magnetische Stimulation (TMS) für das präoperative funktionale Mapping des motorischen Cortex verwendet wird. Der Kopf eines Patienten wird dabei fest und unbeweglich mit einer Kopflehne verbunden, wobei eine Schwenkarm vorgesehen ist, an welchem eine 8-förmige Spule so angebracht ist, dass die Armspitze unter dem Spulenschnittpunkt liegt. Der Arm wird dabei so ausgerichtet, dass die unter dem Schnittpunkt der beiden Spulen liegenden Spitze auf einen bestimmten Bereich zeigt, in welchem ein Strom induziert werden soll.

Aus der US 5,738,625 ist ein Verfahren bekannt, um Nervenzellen magnetisch zu stimulieren.

Die US 5,644,234 beschreibt ein Kernspinresonanz(MR)-Verfahren, wobei die Position einer Mikrospule in einem Objekt ermittelt werden soll.

Aus der WO 98/06342 sind ein Verfahren und eine Vorrichtung zur transcranialen magnetischen Stimulation des Gehirns bekannt, wobei ein etwa halbkugelförmiger von Spulen umwickelter Magnetkern zum Erzeugen eines Stimulationssignals verwendet wird. Mit der beschriebenen Vorrichtung und dem Verfahren soll die Sprachfunktion lokalisiert werden.

Aus Ettinger G.J. et all: "Non-invasive functional brain mapping using registered transcranial magnetic stimulation" Proceedings of the IEEE workshop on mathematical methods in biomedical image analysis (cat. No. 96TB100056), proceedings of the workshop on mathematical methods in biomedical image analysis, San Francisco, CA, USA, 21-22 June 1996, Seiten 32-41, XP002202005 1996, Los Alamitos, CA, USA, IEEE Comput. Soc. Press, USA, ISBN: 0-8186-7367-2 ist ein Verfahren zum Mapping der funktionalen Bereiche des Gehirns unter Verwendung einer Vorrichtung zur transcranialen magnetischen Stimulation bekannt. Die Vorrichtung stimuliert Neuronen durch Erzeugung von fokussierten gepulsten magnetischen Feldern. Dabei wird ein MR-Bild des Objektes vor der TMS-Sitzung aufgenommen und die Aufnahme in gewünschte anatomische Strukturen segmentiert.

Es ist eine Aufgabe der vorliegenden Erfindung Verfahren und Vorrichtungen zur Stimulation bestimmter Bereiche eines Gehirns vorzuschlagen, mit welchen die räumliche Genauigkeit der Stimulation und die Lokalisation bestimmter Hirnbereiche verbessert werden kann.

Diese Aufgabe wird wie in den unabhängigen Ansprüchen definiert gelöst. Vorteilhafte Ausführungsformen ergeben sich aus den Unteransprüchen.

Unter Stimulation wird im Sinne der Erfindung nicht nur das aktive eigentliche Stimulieren eines bestimmten Gehirnbereiches oder -punktes verstanden, sondern auch das Anlegen oder Erzeugen von Signalen, welche eine Funktionsunterdrückung bestimmter Gehirnbereiche verursachen, häufig auch als funktionelle Läsion bezeichnet. Der Begriff "Stimulieren" soll demzufolge so verstanden werden, dass auch ein Blockieren oder Inhibieren von Hirnfunktionen durch bestimmte Stimulationssignale verursacht werden kann. Das Stimulieren oder Inhibieren eines Hirnbereiches erfolgt durch Anlegen bzw. Erzeugen von Signalen im Gehirn durch eine Stimulationsvorrichtung, wie zum Beispiel eine auf den Kopf aufgelegte von Strom durchflossene Spule, um im Gehirn durch Induktion elektrische Signale zu erzeugen, wobei die Signale eine bestimmte Impulsform oder eine Folge von Impulsen mit bestimmten Impulsformen und einer oder mehrerer vorgegebener Frequenzen sein können. Es hat sich gezeigt, dass bei Verwendung höherer Frequenzen zum Beispiel im Bereich von 50 Hz bestimmte Gehirnbereiche blockiert bzw. inhibiert werden können, so dass die von den betroffenen Gehirnbereichen zu erfüllenden Funktionen, wie zum Beispiel die Wahrnehmung eines externen Reizes, nicht mehr durchgeführt werden können. Demgegenüber erfolgt die Stimulation bevorzugt durch einen einzigen magnetischen bzw. dadurch induzierten elektrischen Impuls.

Gemäß einem ersten Aspekt der Erfindung wird für ein Verfahren zum Stimulieren und/oder Inhibieren eines bestimmten Bereichs eines Gehirns, zum Beispiel eines einzelnen Punktes oder eines Clusters bzw. Gitters aus mehreren Punkten, unter Verwendung zum Beispiel einer Induktionsvorrichtung als Stimulationsvorrichtung zunächst die räumliche Struktur des zu untersuchenden Kopfes oder Gehirns aufgenommen, zum Beispiel durch Kernspinresonanz-Verfahren (MRI), Computertomographie (CT), Ultraschall, Röntgen, Videoaufnahmen bei anschließender Rekonstruktion der Hirnoberfläche oder andere bekannte Untersuchungsverfahren. Basierend auf der so erhaltenen räumlichen Struktur des Kopfes bzw. des Gehirns wird ein Simulationsmodell der Gehirnoberfläche erzeugt. Somit wird erfindungsgemäß die Tatsache ausgenutzt, dass zum Beispiel sensorische, motorische, visuelle, auditorische und olfaktorische Funktionen durch an der Hirnoberfläche liegende Bereiche wahrgenommen werden. Da das Gehirn eine unregelmäßige Oberfläche aufweist, wird erfindungsgemäß versucht diese Oberfläche möglichst genau zu simulieren, um basierend auf einem so erzeugten Simulationsmodell eine Stimulation möglichst orthogonal bzw. senkrecht zur Oberfläche eines zu stimulierenden Bereichs durchzuführen. Würde eine Stimulationsvorrichtung, wie beispielsweise eine Spule zur Erzeugung eines Stimulationssignals, wie im Stand der Technik üblich so relativ zur Kopfoberfläche angeordnet, dass das von der Spule erzeugte magnetische Feld möglichst senkrecht zur Kopfoberfläche ist, so könnte es auf Grund der unregelmäßigen Oberfläche des Gehirns dazu kommen, dass ein Oberflächenbereich des Gehirns durch ein magnetisches Feld stimuliert wird, welches nicht senkrecht zur Hirnoberfläche steht und somit auf Grund der schrägen relativen Lage zueinander unbeabsichtigt ein größerer oder auch anderer Bereich des Gehirns stimuliert wird. Allgemein wird durch eine Induktionsvorrichtung wie beispielsweise eine Spule ein magnetisches Feld erzeugt, wodurch ein elektrisches Feld zur Stimulation an bzw. in der Gehirnoberfläche induziert wird. Die Induktionsvorrichtung wird demzufolge unter Verwendung des erfindungsgemäßen Verfahrens so relativ zu einen Kopf bzw. einer Gehirnoberfläche positioniert, dass die Induktionsvorrichtung ein magnetisches Feld erzeugt, welches wenn möglich genau oder nur mit einer relativ geringen Abweichung von zum Beispiel 5 Grad senkrecht auf der Oberfläche des Gehirns steht. Dabei kann es vorkommen, dass die Induktionsvorrichtung nicht orthogonal zur Kopfoberfläche positioniert werden muss. Dies ist insbesondere dann der Fall, wenn der zu stimulierende Bereich an der Oberfläche des Gehirns nicht parallel zur Oberfläche des Kopfes ist. Erfindungsgemäß soll ein bestimmter Bereich des Gehirns bzw. der Hirnoberfläche durch eine Induktionsvorrichtung, bevorzugt durch einen in einer Induktionsvorrichtung fließenden Strom, so stimuliert werden, dass ein Stimulationssignal auf der Oberfläche des Gehirns durch ein möglichst senkrecht auf der Hirnoberfläche stehendes magnetisches Feld erzeugt wird.

Vorteilhaft wird die Gehirnoberfläche durch Polygone modelliert oder approximiert, d. h. basierend auf den durch die Aufnahme der räumlichen Struktur des Gehirns gewonnenen Daten werden Polygone erzeugt, welche als eine Mehrzahl kleinerer Ebenen ein dreidimensionales Modell der Gehirnoberfläche bilden und somit gleichsam die Wände oder Außenbegrenzung der Gehirnoberfläche darstellen. Vorteilhaft wird eine Induktionsvorrichtung erfindungsgemäß dann so positioniert, dass falls eine Stimulation eines bestimmten Bereichs der Hirnoberfläche durchgeführt werden soll, das von der Stimulationsvorrichtung erzeugte magnetische Feld möglichst senkrecht auf dem Polygon oder der simulierten Gehirnoberfläche steht, welche simuliert werden soll. Dabei kann zum Beispiel die Stimulations- oder Induktionsvorrichtung so positioniert werden, dass sie ein maximales magnetisches Feld in etwa in der Mitte eines zur Simulation der Gehirnoberfläche verwendeten Polygons erzeugt.

Bevorzugt wird als Stimulationsvorrichtung ein oder mehrere Elemente verwendet, welche ein elektrisches oder magnetisches Feld erzeugen können, wie zum Beispiel Spulen mit oder ohne Kern.

Vorteilhaft wird ein sogenanntes "Tracking" einer verwendeten Stimulationsvorrichtung durchgeführt, wobei unter Verwendung bekannter Verfahren und Vorrichtungen, wie zum Beispiel reflektierenden am Kopf und an der Stimulationsvorrichtung angebrachten Markern, eine Registrierung der entsprechenden Elemente vorgenommen wird und eine Stimulationsvorrichtung so navigiert, d. h. gesteuert und relativ zur Gehirnoberfläche positioniert wird, dass ein von der Stimulationsvorrichtung erzeugtes magnetisches Feld möglichst senkrecht auf der Oberfläche des dreidimensionalen Simulationsmodells des Gehirns steht und einen maximalen Wert an dem gewünschten Stimulationspunkt annimmt. Verfahren und Vorrichtungen zum Positionieren und Navigieren von Instrumenten relativ zu einer Person sind im Stand der Technik bekannt und werden hier nicht näher beschrieben. Beispielhaft wird auf die in der Prioritätsanmeldung EP 01 114 832.6 der Anmelderin beschriebenen Verfahren verwiesen, wobei die Lehre dieser Patentanmeldung diesbezüglich und insbesondere bezüglich der Erzeugung von Simulationsmodellen einer Induktionsvorrichtung und/oder eines Kopfes in die Offenbarung dieser Anmeldung aufgenommen wird.

Bevorzugt wird die Position einer Stimulationsvorrichtung relativ zu einem gewünschten zu stimulierenden Punkt ermittelt und vorteilhaft angezeigt, so dass eine Person unter Verwendung der so ermittelten relativen Lage von Stimulationsvorrichtung und gewünschtem Stimulationspunkt, die Stimulationsvorrichtung in die gewünschte Position zum Stimulieren des Stimulationspunktes bringen kann. Dabei kann vorteilhaft eine Sperrung der Stimulationsvorrichtung, wie zum Beispiel eine Sperrung des Spulenstroms vorgesehen sein, falls zum Beispiel der Winkel zwischen einem von der Stimulationsvorrichtung erzeugbaren magnetischen Feld um mehr als einen vorgegebenen Winkel von zum Beispiel 5 Grad von der Senkrechten auf die simulierte Gehirnoberfläche, beispielsweise von der Senkrechten auf einem bestimmten Polygon über einem zu stimulierenden Bereich abweicht. Dabei kann beispielsweise ein von der Stimulationsvorrichtung erzeugbares magnetisches und/oder induziertes elektrisches Feld simuliert und angezeigt werden, um so die Richtigkeit der Position der Stimulationsvorrichtung vor dem tatsächlichen Durchführen einer Stimulation zu überprüfen.

Der dreidimensionale Datensatz zum Erzeugen eines Simulationsmodells der Gehirnoberfläche kann in seinem Detaillierungsgrad über die gesamte Hirnoberfläche variieren, um somit beispielsweise größere gleichmäßigere Gehirnbereiche durch eine geringere Anzahl von Polygonen zu modellieren und Hirnbereiche mit größerem Detaillierungsgrad durch eine größere Anzahl an Polygonen zu modellieren.

Das oben beschriebene Verfahren kann sowohl ohne, als auch in Verbindung mit dem in der oben erwähnten Patentanmeldung EP 01 114 823.6 beschriebenen Verfahren verwendet werden, d. h. es kann zum Beispiel auch ein Simulationsmodell des Kopfes und/oder der Induktionsvorrichtung erzeugt und verwendet werden, um eine Stimulationsvorrichtung geeignet zu positionieren. Dabei kann eine Stimulationsvorrichtung, wie beispielsweise eine Induktionsspule, einfach so positioniert werden, dass eine Linie, welche durch die Mitte der Spule hindurchgeht, senkrecht oder unter einem bestimmten Winkel auf dem Simulationsmodell der Gehirnoberfläche stehen soll, um einen gewünschten Punkt zu stimulieren. Ergänzend können aber auch genauere Simulationsmodelle der Induktionsvorrichtung und/oder Simulationsmodelle des Kopfes unter Verwendung zum Beispiel eines Mehr-Schalen-Modells des Kopfes, wie in der EP 01 114 823.6 beschrieben, verwendet werden.

Es ist auch möglich das oben beschriebene und das in der EP 01 114 823.6 beschriebene Verfahren zusammen zu verwenden, d. h. es wird zum Beispiel unter Verwendung des oben beschriebenen Verfahrens die Oberfläche des Gehirns modelliert und dabei werden die elektrischen und magnetischen Eigenschaften der über der Hirnoberfläche liegenden Strukturen gemäß der Lehre der EP 01 114 823.6 berücksichtigt, um die Spule vor einer tatsächlich durchgeführten Stimulation richtig positionieren zu können.

Die Erfindung bezieht sich gemäß einem weiteren Aspekt auf ein Computerprogramm, welches das Verfahren mit einem oder mehreren der oben beschriebenen Schritte durchführt, wenn es in einem Computer geladen wird oder auf einem Computer läuft. Weiterhin bezieht sich die vorliegende Erfindung auf ein Programmspeichermedium oder ein Computerprogrammprodukt mit einem solchen Programm.

Nach einem weiteren Aspekt bezieht sich die Erfindung auf eine Vorrichtung zum Stimulieren eines bestimmten Bereichs eines Gehirns mit einer Stimulations- bzw. Induktionsvorrichtung, wobei eine Aufnahmevorrichtung, wie beispielsweise ein Kernspintomograph vorgesehen ist, um die räumlich Struktur des Kopfes, insbesondere des Gehirns aufzunehmen. Basierend auf den so gewonnenen Daten wird in einer Rechenvorrichtung ein Simulationsmodell zum dreidimensionalen Approximieren bzw. Simulieren der Gehirnoberfläche erzeugt.

Vorteilhaft ist eine Navigationsvorrichtung vorgesehen, welche dazu dient, um die bevorzugt mit Markern versehene Stimulations- bzw. Induktionsvorrichtung so relativ zum Kopf bzw. zur Gehirnoberfläche zu positionieren, dass ein bestimmter Bereich des Gehirns bzw. der Gehirnoberfläche durch einen Impuls der Stimulationsvorrichtung, beispielsweise einen in einer Induktionsvorrichtung fließenden Strom, stimuliert werden kann.

Bevorzugt ist eine Steuervorrichtung, vorteilhaft in Verbindung mit einer Anzeigeeinheit vorgesehen, mit welcher die oben beschriebenen Verfahrenschritte gesteuert und die Positionen zum Beispiel der Stimulationsvorrichtung relativ zum Kopf bzw. zur simulierten Hirnoberfläche und die Stimulationssignale dargestellt werden können.

Die Verwendung des oben beschriebenen Verfahrens und der oben beschriebenen Vorrichtung ermöglichen das Positionieren einer Stimulationsvorrichtung, wie beispielsweise einer Spule auf eine Art, dass ein durch die Stimulationsvorrichtung erzeugtes magnetisches Feld möglichst senkrecht auf die zu stimulierende Gehirnoberfläche trifft, wobei in Nährung beispielsweise die Mittelachse einer Spule so positioniert werden kann, dass diese senkrecht auf einem dreidimensionalen Simulationsmodell der Hirnoberfläche steht, bevor ein oder mehrere Impulse erzeugt werden.

Das oben beschriebene Verfahren und die Vorrichtung können sowohl dazu verwendet werden, einen einzigen Punkt im Gehirn bzw. auf der Hirnoberfläche zu stimulieren, der häufig auch als "Hot Spot" bezeichnet wird. Um jedoch funktionale Bereiche im Gehirn aufzufinden, die für komplexere Funktionen verantwortlich sind, ist es häufig nicht ausreichend eine transcraniale magnetische Stimulation eines einzigen Punktes durchzuführen und die evozierten Potentiale zu verwenden, um die Position zum Beispiel des motorischen Cortex zu ermitteln. Es ist nämlich bekannt, dass bestimmte Gehirnfunktionen nicht von eng umgrenzten Bereichen des Gehirns wahrgenommen werden, sondern sich auf größere Bereiche erstrecken oder verteilen können. Allgemein stellt ein Gyrus, d. h. eine Gehirnwindung, häufig die einzig bekannte "scharfe" Grenze einer Hirnfunktion dar, wobei der Zwischenraum zwischen zwei Hirnwindungen als Sulcus bezeichnet wird. Jedoch können zum Beispiel unterschiedliche motorische Funktionen von unterschiedlichen Positionen auf einem Gyrus oder beispielsweise Sprachfunktionen von verschiedenen Gyri wahrgenommen werden.

Gemäß einem weiteren Aspekt der Erfindung wird ein Verfahren vorgeschlagen, mit welchem mindestens ein bestimmter Punkt des Gehirns oder der Gehirnoberfläche stimuliert wird, wobei mindestens zwei, bevorzugt eine Mehrzahl, wie zum Beispiel 8, 32, 100 oder mehr Reizantworten an einer Person erfasst werden, zum Beispiel indem mehrere Reizerfassungsvorrichtungen an verschiedenen Positionen, wie zum Beispiel an den einzelnen Fingern einer Hand und/oder anderen Muskelgruppen vorgesehen sind, um so durch eine Mehr-Kanal-Aufzeichnung und vorteilhaft eine Mehr-Kanal-Stimulation eine Information darüber zu erhalten, wie sich eine bestimmte Stimulation bzw. ein Stimulationsmuster bei einem oder mehreren Bereichen des Gehirns gleichzeitig oder nacheinander angelegt auf die Muskelgruppen auswirken. Dabei kann gemessen werden welcher Muskel bzw. welche Muskelpartie sich wann und wie stark bewegt, wenn ein bestimmtes Stimulationsmuster angelegt wird. Aus der Zuordnung von Stimulationsmustern zu den über mehrere Kanäle aufgezeichneten Reizantworten kann eine Information über die tatsächliche Zuordnung einer bestimmten Funktion, wie beispielsweise das Erzeugen eines Muskelzuckens zu einer Position oder einem Bereich auf einem Gyrus gewonnen werden. Damit können zum Beispiel mit einem sequentiellen Stimulationsmuster nach einer bestimmten Anzahl von Stimulationsimpulsen aus den gemessenen Reizantworten die funktionellen Areale ermittelt werden. Dieses Verfahren ermöglicht ein schnelleres Lokalisieren funktioneller Areale.

Vorteilhaft wird eine Gitterstruktur zum Festlegen verschiedener Stimulationspunkte auf der Gehirnoberfläche definiert, wobei Stimulationsimpulse an den Kreuzungspunkten der Gitterlinien beispielsweise einzeln und nacheinander an verschiedenen Kreuzungspunkten oder gleichzeitig an mehreren Kreuzungspunkten mit gleicher oder unterschiedlicher Intensität und gleichem oder unterschiedlichem Frequenzmuster angelegt werden können. Bevorzugt erfolgt die Positionierung der Gitterstruktur so, dass das Gitter nur auf einem oder mehreren funktionellen Arealen liegt, wobei die Positionierung automatisch oder semi-automatisch durchgeführt werden kann, d. h. zum Beispiel von einem Computer unter Berücksichtigung des dreidimensionalen Modells des Gehirns positionierte Gitterpunkte können manuell nachjustiert werden. Die Positionierung einer Stimulationsvorrichtung, wie beispielsweise einer Spule, kann auf bekannte Art, wie in der EP 01 114 823.6 beschrieben, oder unter Verwendung der oben beschriebenen Verfahren und Vorrichtungen durchgeführt werden.

Wird beispielsweise eine Stimulation im Bereich des motorischen Cortex durchgeführt, so ist es relativ einfach die Stärke der Reizantwort, in diesem Fall die Stärke eines Muskelzuckens zu messen. Allgemein können auch bei Stimulation anderer Hirnbereiche die Stärken der Reizantworten, zum Beispiel die Helligkeit eines wahrgenommenen Blitzes oder die Lautstärke eines gehörten Tones ermittelt werden.

Dabei kann beispielsweise eine TMS-Spule computergeführt von einem Gitterpunkt zu einem anderen bewegt bzw. navigiert werden und ein Stimulationsimpuls mit einem vorgegebenen Wert oberhalb einer vorgegebenen Reizschwelle kann über die TMS-Spule induziert werden. Es werden immer alle Reizantworten einer bestimmten Stimulation gleichzeitig aufgezeichnet und so kann für die Stimulation eines einzelnen oder auch mehrerer Gitterpunkte gleichzeitig die Reizantwort ermittelt werden.

Vorteilhaft wird ein bestimmter Stimulationsimpuls oder ein Stimulationsmuster mit gleicher Intensität mehrfach angelegt, um beispielsweise eine Mittelwertbildung oder eine andere Auswertung der erfassten Messdaten zu ermöglichen.

Vorteilhaft kann unter Verwendung des oben beschriebenen Verfahrens für jede Reizantwort, d. h. zum Beispiel für das Bewegen eines bestimmten Muskels eine Verteilungsfunktion oder Intensitätsfunktion der Repräsentation der motorischen Steuerung dieses Muskels auf dem motorischen Cortex ermittelt werden. Mit anderen Worten kann durch das gleichzeitige Aufzeichnen von mehreren Reizantworten bei bestimmten Stimulationsmustern eine Zuordnung einer bestimmten Reizantwort, wie beispielsweise eines Muskelzuckens, zu einem bestimmten Hirnbereich ermittelt werden, wobei Hirnbereiche mit einer stärkeren und schwächeren Korrelation zu dieser Reizantwort durch die Verwendung der Lage der zur Stimulation verwendeten Gitterpunkte bestimmt werden können. Die aus den einzelnen Reizantworten gewonnene Intensitäts- oder Empfindlichkeitsfunktion kann beispielsweise als eine dreidimensionale Verteilung über der Hirnoberfläche aufgefasst werden, wobei die Empfindlichkeitsfunktion eines ersten Muskels sich mit der Empfindlichkeitsfunktion eines zweiten Muskels überlappen kann und beispielsweise an einer anderen Position einen Maximalwert annimmt.

Es ist auch möglich eine Stimulationsvorrichtung zu verwenden, bei welcher mehrere Stimulationsimpulse gleichzeitig abgegeben werden können, wobei beispielsweise eine Mehrzahl von Spulen in einer Gitterstruktur so angeordnet ist, dass mehrere Spulen gleichzeitig mit gleichen oder unterschiedlichen Stimulationsimpulsen angelegt werden können, um auf der Gehirnoberfläche mehrere Stimulationsimpulse zu erzeugen, wobei aus den gleichzeitig gemessenen Reizantworten ermittelt werden kann, welche stimulierten Bereiche mit welcher Gewichtung bzw. welcher Übertragungsfunktion für das Auslösen einer bestimmten Reizantwort verantwortlich waren.

Die Erfindung bezieht sich gemäß einem weiteren Aspekt auf ein Computerprogramm, welches das Verfahren mit einem oder mehreren der oben beschriebenen Schritte durchführt, wenn es in einen Computer geladen wird oder auf einem Computer läuft. Weiterhin bezieht sich die vorliegende Erfindung auf ein Programmspeichermedium oder ein Computerprogrammprodukt mit einem solchen Programm.

Weiterhin bezieht sich die Erfindung auf eine Vorrichtung zum Stimulieren mindestens eines Punktes des Gehirns und mindestens zwei Aufzeichnungsvorrichtungen, mit welchen die Reizantworten eines über die Stimulationsvorrichtung induzierten Impulses gemessen werden können.

Gemäß einer ersten Ausführungsform ist die Stimulationsvorrichtung eine computergeführte Stimulationsvorrichtung, welche an verschiedenen Positionen des Kopfes oder allgemein relativ zur Gehirnoberfläche möglichst genau positioniert werden kann, um einen Stimulationsimpuls zu erzeugen.

Gemäß einer weiteren Ausführungsform der Erfindung ist die Stimulationsvorrichtung zusammengesetzt aus einer Mehrzahl einzelner Stimulationsvorrichtungen, wie beispielsweise mehreren Spulen, um gleichzeitig Stimulationsimpulse an verschiedenen Stellen des Gehirns induzieren zu können. Diese Stimulationsvorrichtung kann ebenfalls computergeführt positioniert werden und kann weiterhin beispielsweise so ausgestaltet sein, dass die einzelnen Induktionsvorrichtungen bzw. Spulen beispielsweise auf einer Kugel- oder Ellipsoid-Oberfläche liegen, um so auf einen Kopf aufgelegt werden zu können, wobei dann vorteilhaft die einzelnen Stimulationsvorrichtungen in etwa den gleichen Abstand zur Hirnoberfläche aufweisen sollten. Alternativ kann der Abstand einzelner Stimulationsvorrichtungen variiert werden, um die Parameter eines Stimulationsimpulses zu variieren.

Vorteilhaft ist eine Steuervorrichtung, bevorzugt in Verbindung mit einer Anzeigevorrichtung vorgesehen, um mindestens einen der oben beschriebenen Verfahrenschritte auszuführen und verschiedene Informationen, wie beispielsweise die Positionierung einer Induktionsvorrichtung relativ zum Gehirn, die simulierte Gehirnoberfläche, ein Simulationsmodell des induzierten Impulses, oder ähnliches anzuzeigen.

Die Stimulationsimpulse, welche an einer oder mehreren Stellen der Gehirnoberfläche angelegt werden, können als ein Eingangsvektor aufgefasst werden, dessen einzelne Elemente aus den Stimulationsimpulsen bestehen. Die an mehreren Stellen gemessenen Reizantworten können als Ausgangsvektor eines Systems aufgefasst werden, welches durch das Gehirn dargestellt wird und in Form einer Matrix beschrieben werden kann, um aus dem erwähnten Eingangsvektor den Ausgangsvektor zu erhalten.

Gemäß einem weiteren Aspekt der vorliegenden Erfindung wird die oben beschriebene TMS-Technologie zum Lokalisieren verschiedener Bereiche des Gehirns, wie beispielsweise des Sprach-Cortex, des visuellen Cortex, des sensorischen Cortex, des auditorischen Cortex oder des olfaktorischen Cortex verwendet. Dabei wird bevorzugt ein automatisches oder automatisiertes System verwendet, um eine Karte der entsprechenden Hirnfunktionen zu erzeugen.

Allgemein besteht das Problem, dass komplexere Hirnfunktionen nicht in einem eng umgrenzten Bereich lokalisiert sind, sondern durch ein Zusammenspiel mehrerer Hirnbereiche (Cluster) realisiert werden. Es können Impulse zum Stimulieren oder Inhibieren verwendet werden, um beispielsweise eine Reizantwort auf einen Stimulationsimpuls oder eine funktionale Läsion, d. h. kurzzeitige Funktionsunterdrückung zu erhalten, um basierend auf diesen Informationen die Kartierung, d. h. funktionale Zuordnung von Hirnbereichen zu bestimmten Funktionen durchführen zu können. Stimulationsimpulse führen im Allgemeinen dazu, dass bestimmte Aktionen wie beispielsweise ein Muskelzucken ausgelöst werden, oder Sinneseindrücke, wie beispielsweise das optische Wahrnehmen eines Blitzes, das Hören eines bestimmten Tones, das Riechen eines bestimmten Geruchs oder beispielsweise das Wahrnehmen einer taktilen Sensation, wie beispielsweise eines Stechens hervorgerufen werden. Wird ein Inhibitionssignal zur Erzeugung funktionaler Läsionen erzeugt, was beispielsweise durch das Anlegen hochfrequenter Impulse erfolgen kann, so kann über an einer Testperson festgestellte Funktionsausfälle des entsprechenden Hirnbereiches ermittelt werden, welche Hirnfunktion gerade blockiert ist. Dabei kann beispielsweise ein bestimmter Bereich des Sehfeldes blockiert werden, ein Frequenzbereich nicht mehr gehört werden, bestimmte Sprachfunktionen unterdrückt werden, sensorische Eindrücke wie beispielsweise ein Stechen oder bestimmte Gerüche nicht mehr wahrgenommen werden.

Es können einer Person bestimmte Reize, wie beispielsweise optische, akustische, sensorische oder olfaktorische Eindrücke auf herkömmliche Weise dargeboten werden, um der Person die Möglichkeit zu geben diese Reize mit Reizen zu vergleichen, welche durch oben beschriebene Stimulationsverfahren und Stimulationsvorrichtungen erzeugt wurden. Dabei kann ein iteratives Verfahren durchgeführt werden, bis eine möglichst genaue Übereinstimmung zwischen natürlichem Sinneseindruck und durch TMS hervorgerufenem Sinneseindruck vorliegt.

Bezüglich des Anlegens bzw. Erzeugens von Stimulationssignalen wird auf vorher beschriebene Verfahren und Vorrichtungen verwiesen.

Zum Auffinden und Untersuchen des visuellen Cortex kann eine Vorrichtung, wie beispielsweise ein Projektor oder eine spezielle Brille vorgesehen sein, über welche die bekannte Gesichtsfeldperimetrie, auch als Goldman-Perimeter bekannt, zum Beispiel durch Linien dargestellt wird. Wenn ein TMS-Stimulationssignal im Bereich des visuellen Cortex erzeugt wird, wird eine Person einen optischen Eindruck, wie beispielsweise einen kurzen Blitz wahrnehmen oder, je nach Art des Signals, auch einen bestimmten Bereich eines dargestellten Bildes nicht mehr erkennen. Der Bereich der Wahrnehmung des durch das TMS-Signal erzeugten Blitzes oder der Verlust eines Bildbereiches in der Gesichtsfeldperimetrie kann mit dem Bereich auf dem visuellen Cortex korelliert werden, bei welchem die Stimulationsimpulse erzeugt wurden, wodurch eine Zuordnung bestimmter Gesichtsfeldbereiche zu bestimmten Arealen des visuellen Cortex vorgenommen werden kann.

Zur Untersuchung des auditorischen Cortex kann eine Vorrichtung zur Erzeugung von Tönen, wie beispielsweise ein Lautsprecher oder Kopfhörer vorgesehen sein, um Töne direkt über die Luft zu dem Ohr einer Person zu übertragen (Luftleitung). Es ist alternativ auch möglich einen elektromechanischen Vibrator zu verwenden, welcher auf einem Knochen in der Nähe des Ohres aufgesetzt wird, um einen akustischen Eindruck zu erzeugen (Knochenleitung). Dabei wird ein Ton bei einer konstanten Frequenz erzeugt, welcher in Abhängigkeit von TMS-Stimulationsimpulsen entweder unterdrückt wird oder als Referenz für einen durch TNS induzierten Klangeindruck verwendet werden kann. Wird eine TMS-Stimulationsvorrichtung über verschiedene Bereiche des auditorischen Cortex bewegt, kann eine Kartierung des auditorischen Cortex zur Darstellung der Wahrnehmung verschiedener Frequenzbereiche gewonnen werden.

Die oben für den visuellen und auditorischen Cortex beschriebenen Anordnungen können auch zur Untersuchung des Sprach-Cortex verwendet werden. Dabei können beispielsweise über einen Monitor und/oder Kopfhörer einer Person bestimmte optische und Schallsignale dargeboten werden. Bevorzugt ist ein Mikrophon vorgesehen, um von der Person gesprochene Sprache aufzunehmen. Es werden verschiedene Sprach-Paradigmen verwendet, wie zum Beispiel eine Benennungsaufgabe, bei welcher eine Person einen gezeigten Gegenstand benennen muss, eine Leseaufgabe, eine Assoziationsaufgabe oder eine Interpretationsaufgabe. Dabei werden die von der Person gegebenen Antworten gesammelt und von einem Computersystem verarbeitet, um eine Information bezüglich der Zuordnung verschiedener Sprachfunktionen auf dem Sprach-Cortex zu erhalten. Es ist zum Beispiel möglich, dass ein Computersystem basierend auf den von der Person gegebenen Antworten eine oder mehrere TMS-Stimulationsvorrichtungen automatisch unter Verwendung der oben beschriebenen Verfahren und Vorrichtungen an verschiedenen Stellen positioniert, um so möglichst automatisiert den Sprach-Cortex untersuchen zu können.

Bei der Untersuchung des Sprach-Cortex ist zu beachten, dass anders als beispielsweise beim motorischen Cortex, meistens keine klare Zuordnung von Stimulationsimpulsen zur Reizantwort möglich ist, da die verschiedenen für das Sprachempfinden bzw. Sprachverhalten erforderlichen Funktionen über mehrere Bereiche des Gehirns verteilt sind.

Zur Untersuchung des sensorischen Cortex kann einer Person beispielsweise ein Abbild des menschlichen Körpers vorgelegt werden, wobei eine Person auf Bereiche des Abbildes zeigen soll, in welchem sie durch TMS-Stimulation verursacht einen Sinneseindruck hat. Alternativ kann eine Person auch auf den jeweiligen Teil des eigenen Körpers zeigen oder sprachlich mitteilen, wo gerade ein Sinneseindruck hervorgerufen wurde. Ebenso kann auch durch Inhibition ein sensorischer Eindruck blockiert werden, d. h. eine Versuchsperson fühlt zum Beispiel bei Anlegen von entsprechenden TMS-Signalen keinen sensorischen Reiz, wie beispielsweise einen Stich mehr.

Zur Untersuchung des olfaktorischen Cortex kann einer Person eine Testserie an Düften angeboten werden. Anschließend wird ein TMS-Stimulus an bestimmten Bereichen des olfaktorischen Cortex angelegt. Eine Person kann den über die Nase wahrgenommenen Geruch mit dem über das TMS-Stimulationssignal im Gehirn erzeugten Geruch vergleichen und beispielsweise durch mehrmaliges Wiederholen des Verfahrens iterativ bestimmen, welcher tatsächlich wahrgenommene Geruch am besten dem über TMS erzeugten Geruch entspricht, um so eine Kartierung des olfaktorischen Cortex durchzuführen.

Die Erfindung bezieht sich gemäß einem weiteren Aspekt auf ein Computerprogrammprodukt, welches das Verfahren mit einem oder mehreren der oben beschriebenen Schritte durchführt, wenn es in einen Computer geladen wird oder auf einem Computer läuft. Weiterhin bezieht sich die vorliegende Erfindung auf ein Programmspeichermedium oder ein Computerprogrammprodukt mit einem solchen Programm.

Eine erfindungsgemäße Vorrichtung zum Bestimmen der Funktion eines bestimmten Bereichs des Gehirns weist mindestens eine Vorrichtung zum Stimulieren und/oder Inhibieren mindestens eines bestimmten Bereichs des Gehirns und eine Vorrichtung zur Erzeugung eines visuellen und/oder akustischen und/oder sensorischen und/oder olfaktorischen Sinneseindruckes auf. Insbesondere soll die Vorrichtung so ausgestaltet sein, dass sie zur Durchführung der oben beschriebenen Verfahrensschritte geeignet ist.

Erfindungsgemäß soll mit den oben beschriebenen Verfahren und Vorrichtungen die exakte Stimulation einzelner Hirnareale bevorzugt unter Verwendung von bekannten Navigationsverfahren zum Beispiel mit passiven Markern, die Erfassung der Reizantwort und falls erforderlich die Modifikation von Stimulationsmustern für eine erneute Stimulation automatisch durchgeführt werden. Somit kann beispielsweise die Funktion eines bestimmten Gehirnbereiches einfach bestimmt werden, indem eine Stimulationsvorrichtung automatisch und bevorzugt navigiert an einer zu untersuchenden Person positioniert wird, um automatisch erzeugte Stimulationsmuster an ein oder mehreren Punkten sequentiell und/oder gleichzeitig an bestimmten Bereichen des Gehirns zu erzeugen, wobei eine Messung der Reizantwort oder Reizantworten erfolgt. Dies kann entweder automatisch zum Beispiel durch auf Muskeln aufgebrachte Sensoren erfolgen, oder durch Interaktion mit der zu untersuchenden Person, welche zu bestimmten Sinneseindrücken befragt wird oder von selbst Aussagen hierzu machen soll, gegebenenfalls durch einen Vergleich mit einem Referenzreiz oder durch das Beobachten der Versuchsperson beim Lösen von bevorzugt automatisch gestellten Aufgaben, wie zum Beispiel bei der Untersuchung des Sprach-Cortex.

Obwohl die Erfindung anhand von mehreren Aspekten beschrieben wurde, können die einzelnen Aspekte der Erfindung auch in Kombination miteinander verwendet werden, d. h. es können beispielsweise dreidimensionale Simulationsmodelle der Gehirnoberfläche bei der Stimulation eines oder mehrerer Bereiche des Gehirns und automatischer Erfassung einer oder mehrerer Reizantworten verwendet werden, wobei beispielsweise automatisch die funktionelle Verteilung des Sprach-Cortex dadurch ermittelt wird, dass über einen Computerbildschirm bestimmte Aufgaben gestellt werden, welche unterschiedliche Sprachfunktionen verschiedener Bereiche des Gehirns ansprechen und die Lösung der gestellten Aufgaben automatisch zum Beispiel durch das Sprechen in eine Mikrofon oder die Eingabe in eine Tastatur erfasst wird. Dabei kann in Abhängigkeit von den so gewonnenen Ergebnissen zum Beispiel eine Neu-Positionierung einer oder mehrerer Stimulationsvorrichtungen erfolgen und/oder ein angelegter Stimulationsimpuls zum Stimulieren und/oder Inhibieren verändert werden, worauf der gleiche oder ein anderer Test durchgeführt wird, um so automatisch funktionelle Gehirnareale lokalisieren zu können. Allgemein kann die Erfindung auch zur semiautomatischen Lokalisation bestimmter Gehirnareale verwendet werden, wobei beispielsweise eine Aufsichtsperson in den Testablauf eingreift und bestimmte Testabläufe verändern kann.

Die Erfindung wird nachfolgend an Hand von Ausführungsbeispielen unter Bezugnahme auf die beiliegenden Zeichnungen beschrieben werden. Es zeigen:
- Figur 1: die schematische Verteilung der Hirnfunktionen auf dem Gehirn;
- Figur 2: schematisch die Verteilung der motorischen Funktionen auf dem Gyrus;
- Figur 3A: ein Testmuster zum Vorgeben von Stimulationsorten nach dem Stand der Technik; und
- Figur 3B: die Abweichung der Orte der tatsächlichen Stimulationsimpulse von den vorgegebenen Stimulationsorten aufgrund von Positionierungsungenauigkeiten;
- Figur 4A: MRI-Schnittbilder einer Gehirnaufnahme; und
- Figur 4B: eine erfindungsgemäß approximierte Gehirnoberfläche mit Stimulationspunkt;
- Figur 5: schematisch eine Gitterstruktur zum Erzeugen von Stimulationsmustern auf der Gehirnoberfläche;
- Figur 6A und 6B: Verteilungsfunktionen auf der Gehirnoberfläche für das in Figur 5 beispielhaft gezeigte Gitter für Reizantworten an zwei verschiedenen Muskeln;
- Figur 7A: eine schematische Darstellung von Stimulationspunkten C3, C4 und C5 in einer Gitterstruktur;
- Figur 7B: die für zwei verschiedene Muskeln erhaltenen Reizantworten für die Stimulation an den Punkten C3, C4 und C5; und
- Figur 7C: eine schematische Darstellung einer Verteilungsfunktion der Empfindlichkeit zweier Muskeln bezüglich einer Stimulation an den Punkten C3, C4 und C5;
- Figur 8A: eine Versuchsanordnung zur Untersuchung des visuellen Cortex;
- Figur 8B: eine Darstellung der Gesichtsfeldperimethrie;
- Figur 9: eine schematische Darstellung der neuronalen Verschaltung des virtuellen Systems; und
- Figur 10: eine schematische Darstellung der für Sprache wichtigen Gehirnbereiche.

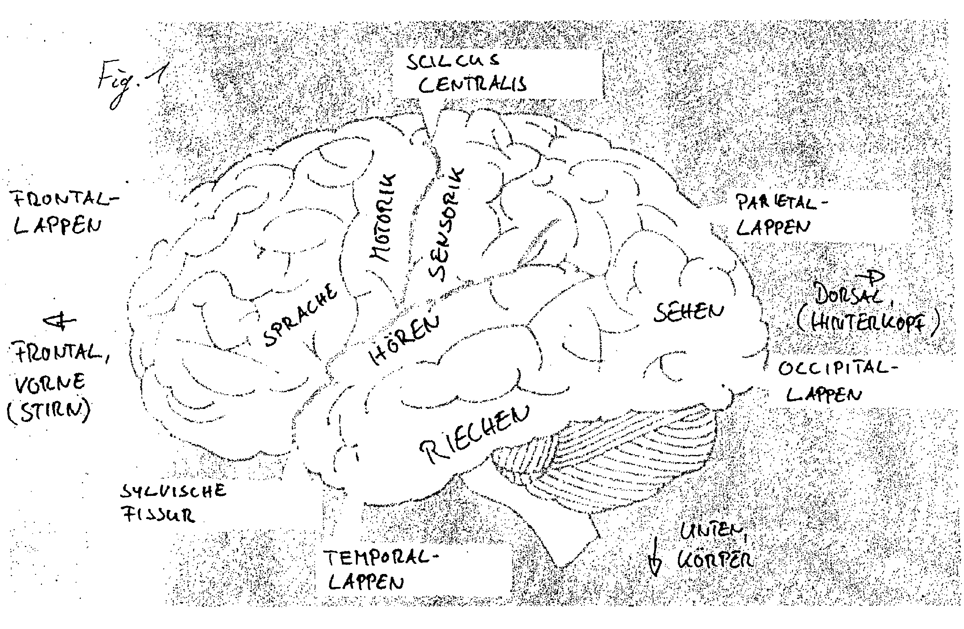

Figur 1 zeigt schematisch die über verschiedene Bereiche des Gehirns verteilten Funktionen, wie zum Beispiel den visuellen, auditorischen, olfaktorischen, motorischen, sensorischen und Sprach-Cortex. Diese Grobeinteilung der Hirnfunktion ist im Wesentlichen bei allen Menschen gleich und kann als erste Näherung zum erfindungsgemäßen Positionieren einer Stimulationsvorrichtung und zum Stimulieren eines bestimmten Hirnbereichs verwendet werden.

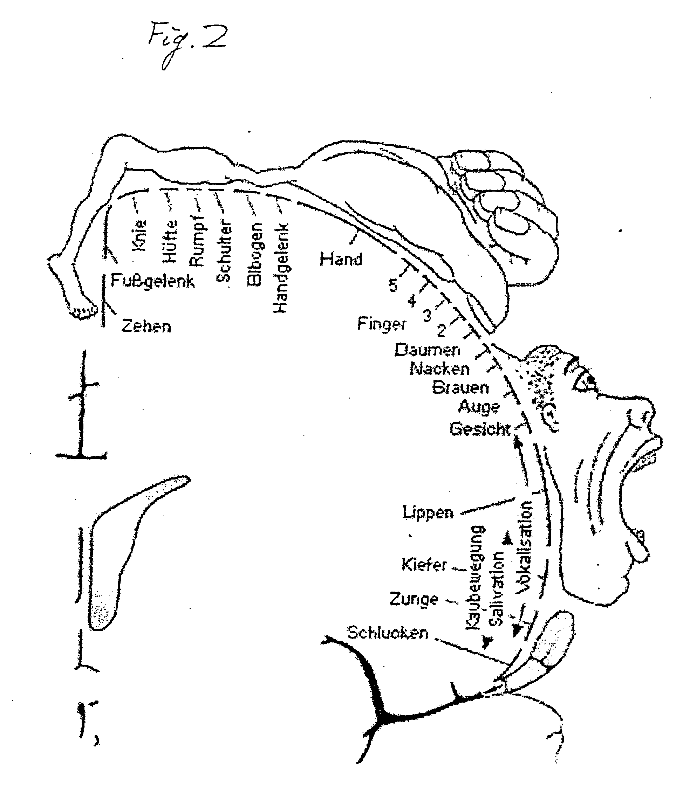

Figur 2 zeigt beispielhaft die auf dem Gyrus precentralis angeordneten motorischen Funktionen mit ihrer Zuordnung zu den jeweiligen Muskeln.

Es sind bereits vielfach Untersuchungen durchgeführt worden, um die Hirnfunktionen grob kartieren zu können, wie in den Figuren 1 und 2 gezeigt. Solche Grobunterteilungen können jedoch nur als Anhaltspunkt für eine weitere exakte kortikale Kartographie verwendet werden, welche wie oben beschrieben erfindungsgemäß durchgeführt wird und zum Beispiel zum möglichst exakten Definieren der Funktion eines bestimmten Gehirnbereiches verwendet werden kann.

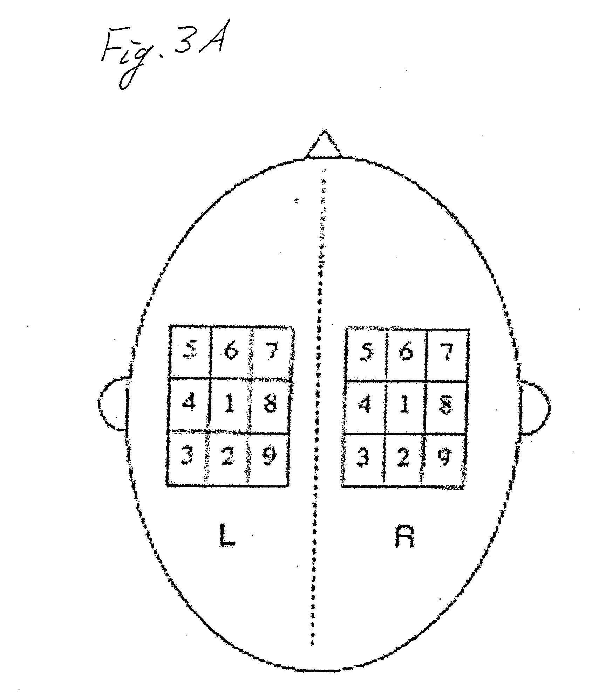

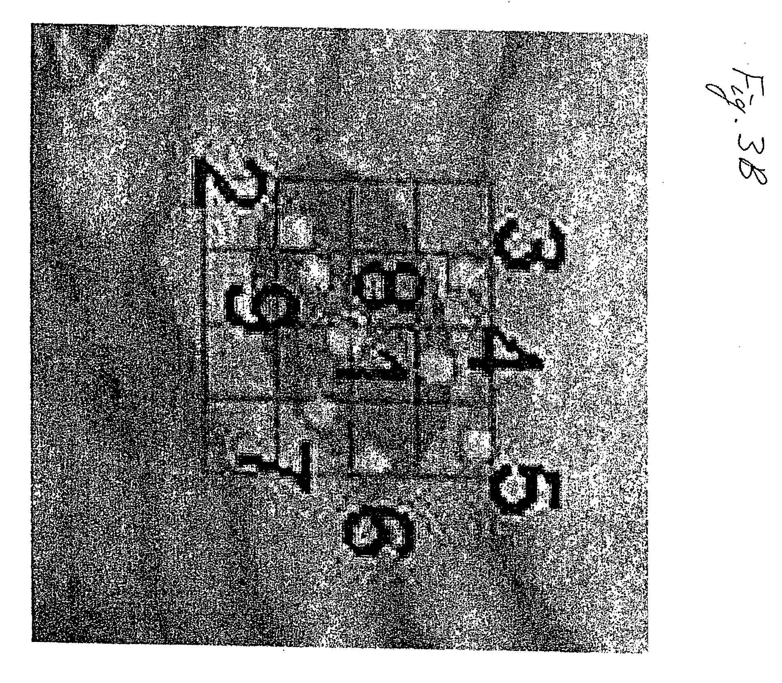

Figur 3A zeigt für die linke und rechte Hirnhälfte jeweils mit 1 bis 9 bezeichnete Positionen zum Positionieren einer Stimulationsvorrichtung. Wird die Stimulation mit einem nach dem Stand der Technik bekannten Verfahren durchgeführt, so tritt beispielsweise die in Figur 3B gezeigte tatsächliche Verteilung der Stimulationspunkte 1 bis 9 auf der Hirnoberfläche auf, was eine unmittelbare Folge der Ungenauigkeit beim bisherigen Positionieren der Stimulationsvorrichtung ohne Positionskontrolle, zum Beispiel über die Darstellung der Gehirnoberfläche und der ungleichmäßigen Struktur der Gehirnoberfläche ist, d. h. die Gehirnoberfläche verläuft nicht exakt parallel zur Oberfläche des Kopfes.

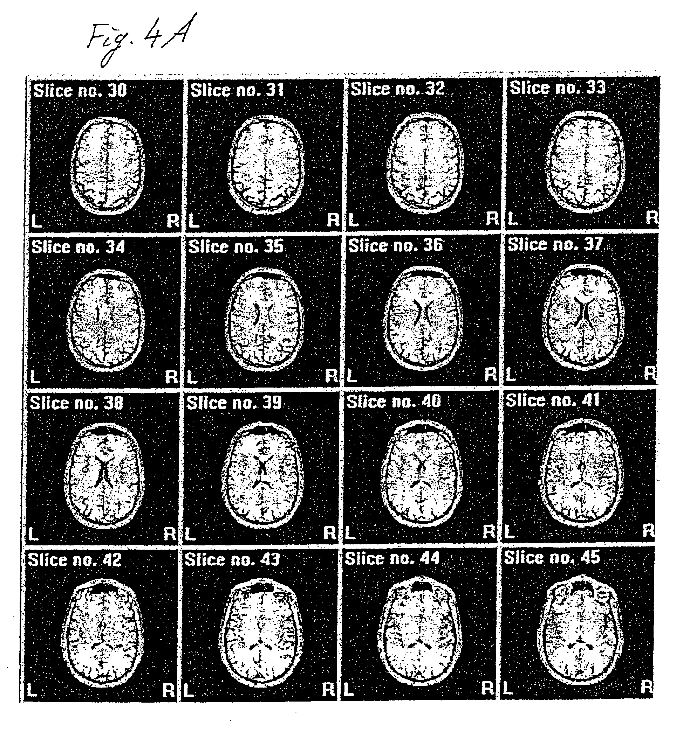

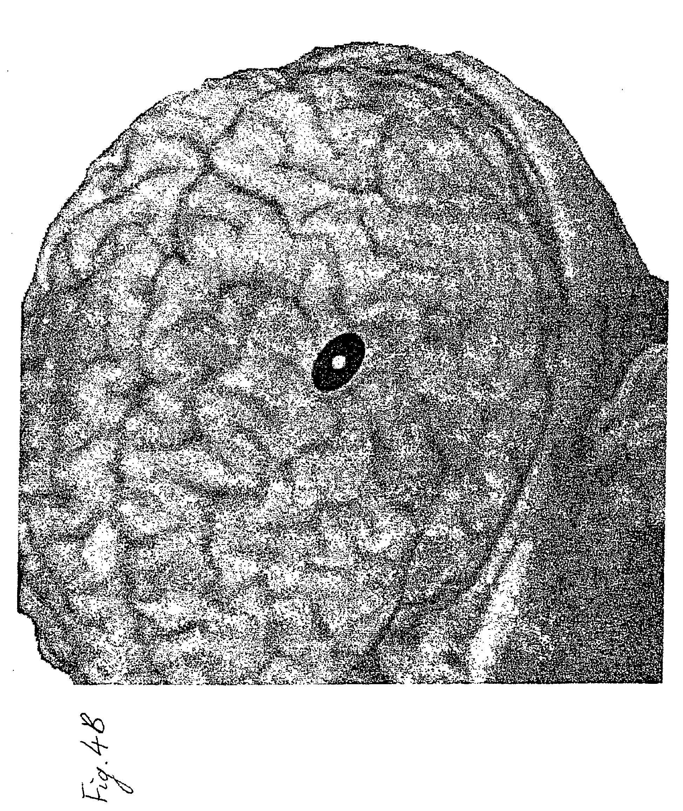

Erfindungsgemäß wird ein zum Beispiel durch Computertomographie oder Kernspinresonanz gewonnener Datensatz aus einer Aufnahme eines Kopfes bzw. eines Gehirns verwendet, wie in Figur 4A gezeigt, um das in Figur 4B gezeigte dreidimensionale Modell bestehend aus einer Vielzahl von Polygonen zur Approximation und dreidimensionalen Beschreibung der Gehimoberfläche zu generieren. Basierend auf dem in Figur 4B gezeigten dreidimensionalen Modell der Hirnoberfläche kann zur Stimulation eines bestimmten in Figur 4B gezeigten Bereiches der Hirnoberfläche die geeignete Position der Stimulationsvorrichtung, wie beispielsweise einer Spule, berechnet werden. Dabei wird zunächst der zu stimulierende Bereich der Hirnoberfläche bestimmt. Das diesen Bereich beschreibende Flächenelement oder ein Mittelwert aus mehreren diesen Bereich beschreibenden Flächenelementen wird verwendet, um möglichst zentral eine auf diesem Bereich senkrecht stehende nach außen weisende Linie zu bestimmen, welche beispielsweise durch die Mittelachse einer Induktionsspule gehen soll, um die Spule so zu Positionieren, dass ein durch die Spule fließender Strom das maximale magnetische Feld an der Oberfläche des so bestimmten Bereiches erzeugt und so durch Induktion ein Stimulationsimpuls in dem gewünschten Oberflächenbereich des Gehirns erzeugt wird. Dabei kann es auf Grund der unregelmäßigen Struktur der Hirnoberfläche vorkommen, dass die beste Position einer Spule nicht senkrecht auf der Kopfoberfläche, sondern schräg dazu steht, um eine möglichst exakte Stimulation eines kleinen Bereichs der Hirnoberfläche durchzuführen.

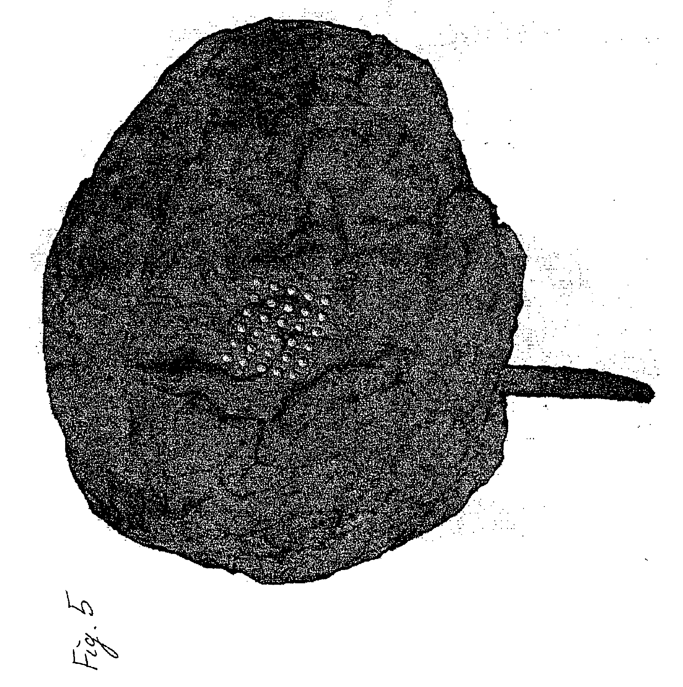

Figur 5 zeigt ein erfindungsgemäß zur Untersuchung eines Gehirnbereichs verwendetes Feld, wobei bei den gezeigten Punkten Stimulationsimpulse sequentiell und/oder parallel erzeugt werden.

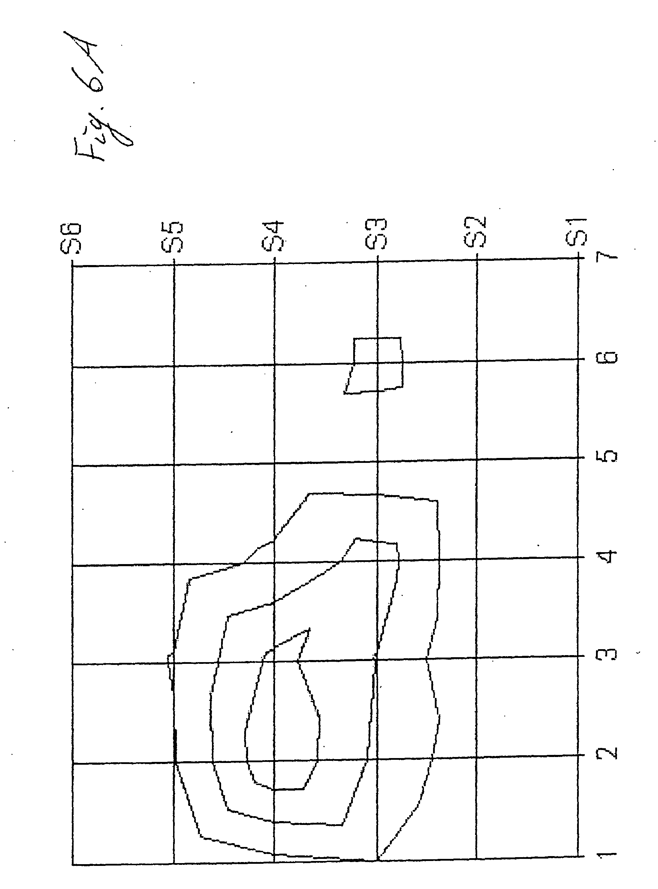

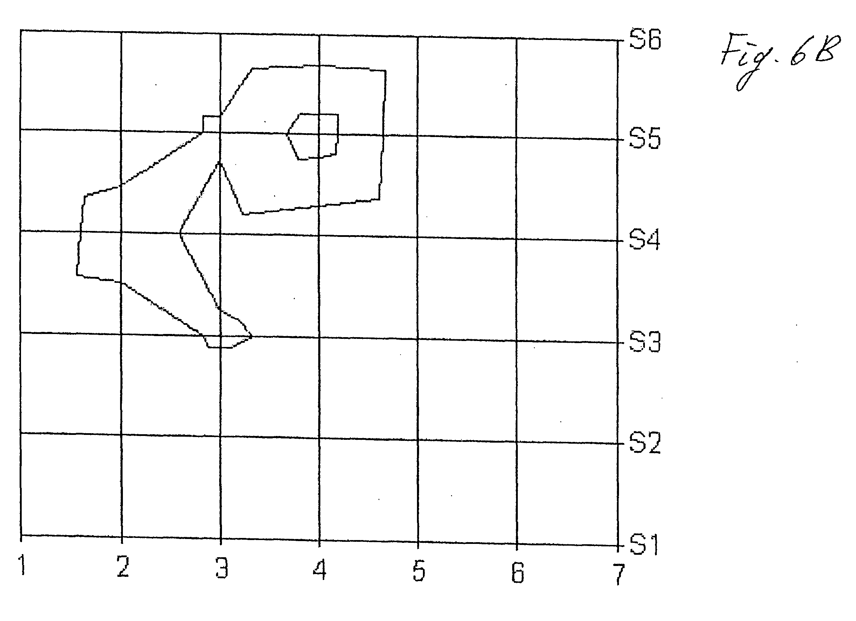

Die Figuren 6A und 6B zeigen die Intensitäten von Reizantworten zweier verschiedener Muskeln in Abhängigkeit vom Ort des oder der Stimulationsimpulse. So kann zum Beispiel aus Figur 6A gesehen werden, dass ein erster Muskel das maximale Ansprechverhalten zeigt, wenn er etwa bei dem mit 2/S4 und 3/S4 gekennzeichneten Bereichen der Hirnoberfläche stimuliert wird, während für einen zweiten Muskel, wie in Figur 6B gezeigt, die maximale Reizantwort bei einer Stimulation bei dem Punkt 4/S5 auftrat. Zur Untersuchung können die einzelnen Stimulationspunkte sowohl sequentiell als auch parallel stimuliert werden, wobei erfindungsgemäß mehrere Muskeln gleichzeitig überwacht werden, um aus der so erhaltenen Mehrkanalaufzeichnung auf die Verteilung der Hirnfunktionen bzw. die Zuordnung einzelner motorischer Funktionen zu einzelnen Gehirnbereichen rückschließen zu können.

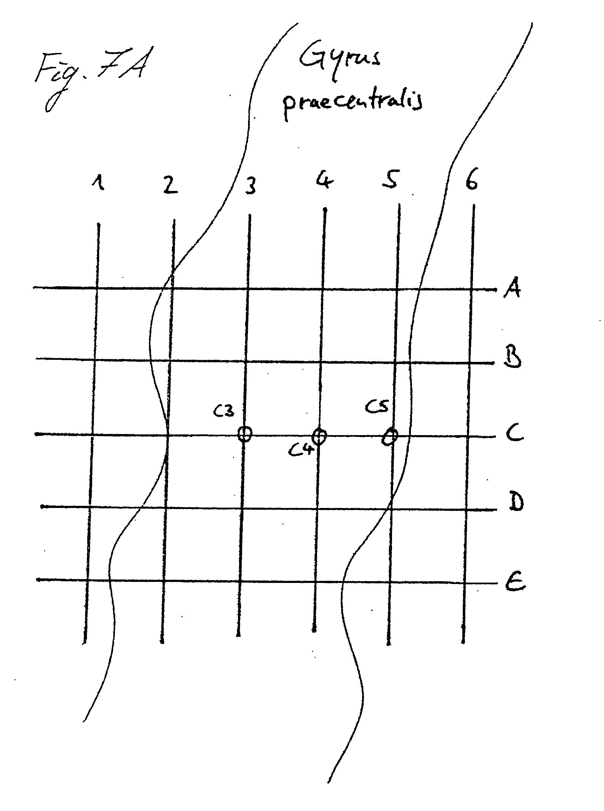

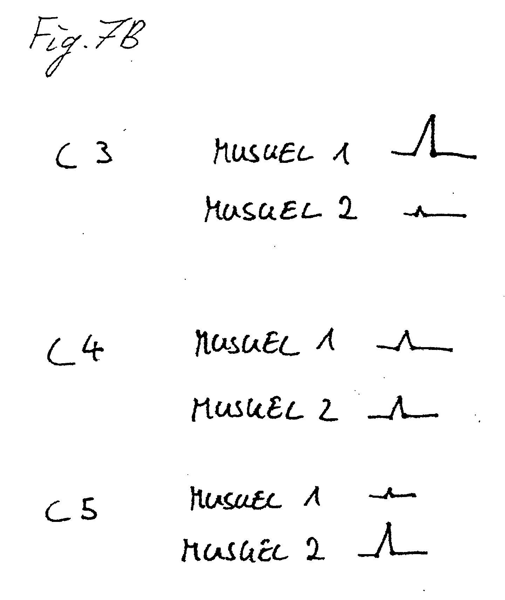

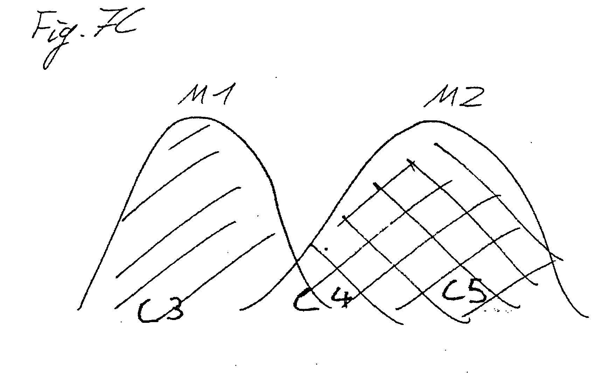

Figur 7A zeigt schematisch eine Gitterstruktur, wobei an den Punkten C3, C4 und C5 Stimulationssignale an die Hirnoberfläche angelegt werden. Für vorgegebene Stimulationssignale an diesen Punkten ergeben sich, wie in Figur 7B gezeigt, für zwei verschiedene Muskeln und unterschiedlich starke Reizantworten. Basierend auf diesen verschieden starken Reizantworten kann eine Verteilungsfunktion oder Empfindlichkeitsfunktion der Hirnoberfläche bezüglich dieser Muskeln und erstellt werden, wie schematisch in Figur 7C gezeigt. Mit anderen Worten kann der Muskel M1 am besten etwa beim Punkt C3 stimuliert werden, während der Muskel M2 am besten beim Punkt C5 stimuliert werden kann.

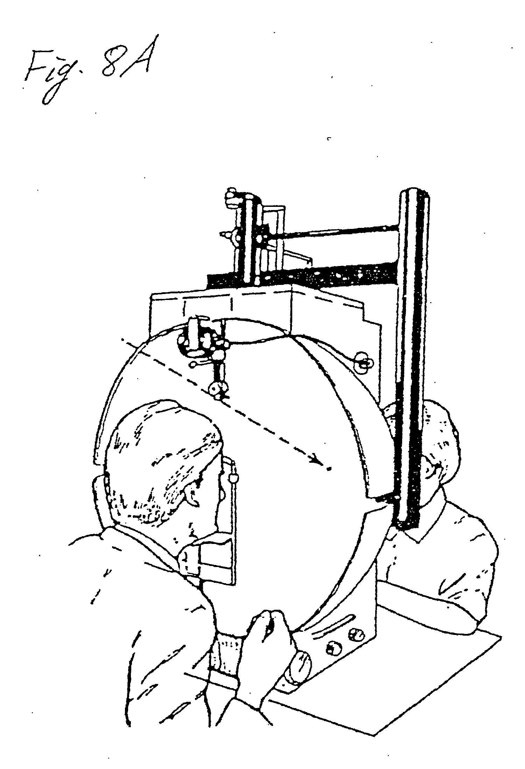

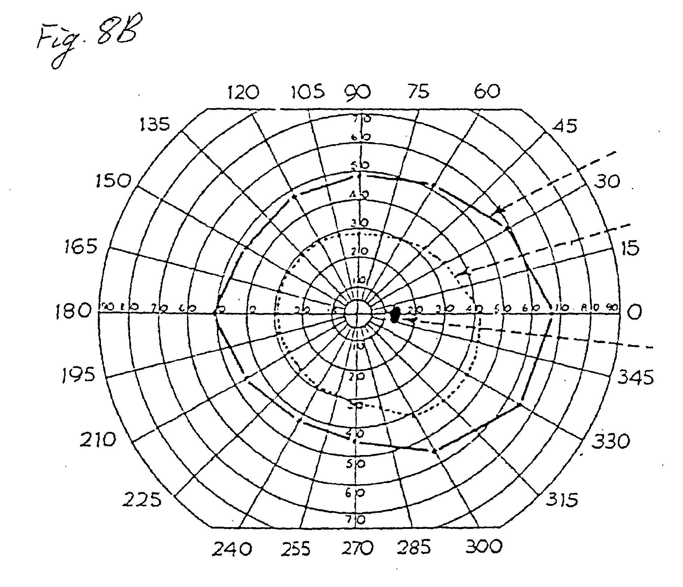

Figur 8A zeigt eine Versuchsanordnung zur Untersuchung des visuellen Cortex, wobei eine Versuchsperson einen bestimmten Punkt fixieren soll. Wie in Figur 8B beispielhaft für das rechte Auge gezeigt, gibt es im Gesichtsfeld rechts neben dem Fixierpunkt einen blinden Punkt, an dem keine optischen Reize wahrgenommen werden können. Gestrichelt ist das zentrale Gesichtsfeld und mit durchgezogener Linie das periphere Gesichtsfeld eingezeichnet. Eine Versuchsperson, welche über TMS-Stimulationssignale induziert bekommt, soll angeben, in welchem Bereich des Gesichtsfeldes das Stimulationssignal einen Blitz hervorruft oder welcher Bereich des Sichtfeldes durch ein Inhibitionssignal nicht mehr wahrgenommen werden kann, um so den visuellen Cortex kartographieren zu können, d. h. einzelnen Strukturen auf dem visuellen Cortex die Sehfelder des linken und rechten Auges zuordnen zu können.

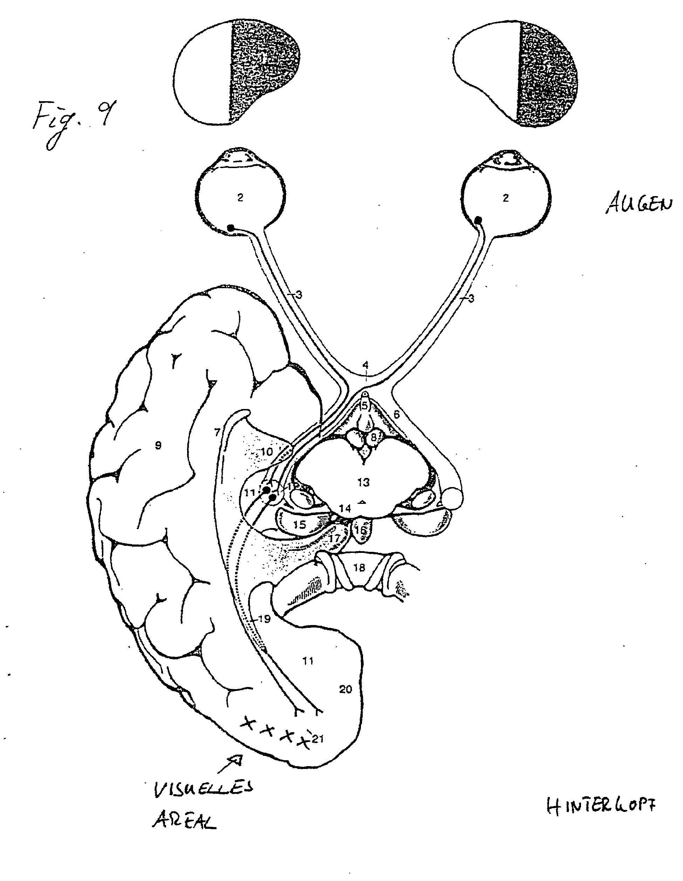

Figur 9 zeigt schematisch die neuronale Verschaltung des visuellen Systems in einer Ansicht von unten. Dabei ist das visuelle Areal eingezeichnet, an welchem die oben erwähnten TMS-Untersuchungen durchgeführt werden.

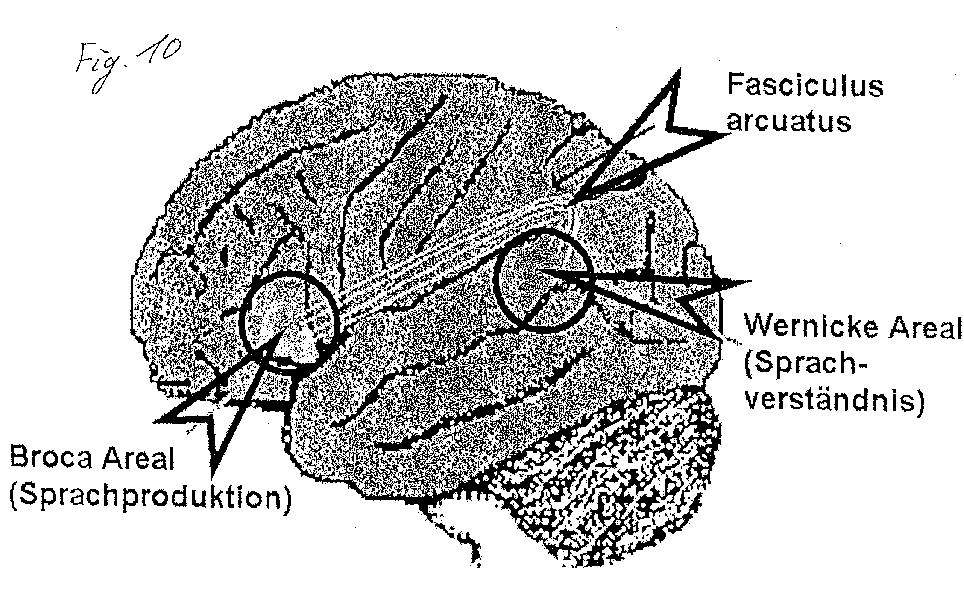

Figur 10 zeigt schematisch die für die Sprachfunktionen wichtigen Bereiche des Gehirns . Das Broca-Areal dient zur Generierung von Sprache, das Wernicke-Areal zur Interpretation von Gesprochenem. Diese Areale sind miteinander vernetzt. Im Gegensatz zu den anderen funktionellen Arealen ist die Sprache meist nur auf einer Gehirnhälfte lokalisiert, hier besteht jedoch eine sehr hohe individuelle Varianz.

## Patentansprüche

1. Verfahren zum Positionieren und Ansteuern einer Stimulationsvorrichtung relativ zu einem Punkt oder Bereich des Gehirns, wobei:
a) die räumliche Struktur des Gehirns aufgenommen wird;
b) ein dreidimensionales Simulationsmodell der Gehirnoberfläche aus der Aufnahme der räumlichen Struktur des Gehirns erzeugt wird;
c) die Stimulationsvorrichtung relativ zu dem Gehirn unter Verwendung des dreidimensionalen Simulationsmodells der Gehirnoberfläche so angeordnet wird, dass der mindestens eine Punkt oder Bereich des Gehirns durch die Stimulationsvorrichtung stimuliert werden kann und dass das von der Stimulationsvorrichtung erzeugbare magnetische Feld senkrecht steht auf dem Simulationsmodell der Gehimoberfläche oder das von der Stimulationsvorrichtung erzeugte magnetische Feld senkrecht steht auf der Gehirnoberfläche an oder über dem zu stimulierenden Bereich oder einen vorgegebenen Raumwinkel dazu aufweist; und
d) ein Stimulationssignal nur freigegeben oder erzeugt wird, wenn die Abweichung der errechneten Position der Stimulationsvorrichtung von der tatsächlichen Position der Stimulationsvorrichtung kleiner als ein vorgegebener Wert ist oder einen vorgegebenen Winkelbereich einhält.

2. Verfahren nach Anspruch 1, wobei die Aufnahme der räumlichen Struktur des Gehirns durch ein Kernspinnresonanz-, Computertomographie-, Röntgen- oder Ultraschall-Verfahren durchgeführt wird.

3. Verfahren nach einem der vorhergehenden Ansprüche, wobei das Simulationsmodell der Gehirnoberfläche durch eine Mehrzahl von Polygonen gebildet wird.

4. Verfahren nach einem der vorhergehenden Ansprüche, wobei eine oder mehrere Spulen als Stimulationsvorrichtung verwendet werden.

5. Verfahren nach einem der vorhergehenden Ansprüche, wobei die Stimulationsvorrichtung zu der berechneten Position für die gewünschte Stimulation navigiert wird

6. Verfahren nach einem der vorhergehenden Ansprüche, wobei die Positionsdaten der Stimulationsvorrichtung und/oder des Gehirns und/oder das an der Hirnoberfläche induzierbare elektrische Feld angezeigt werden.

7. Verfahren nach einem der vorhergehenden Ansprüche, wobei ein Simulationsmodell der Induktionsvorrichtung und/oder des Gehirns, insbesondere ein Mehr-Schalen-Modell verwendet wird.

8. Computerprogramm, welches das Verfahren nach einem der vorhergehenden Ansprüche ausführt, wenn es in einen Computer geladen ist oder auf einem Computer läuft.

9. Programmspeichermedium oder Computerprogrammprodukt mit dem Programm nach Anspruch 8.

10. Vorrichtung zum Stimulieren und/oder Inhibieren mindestens eines Punktes oder Bereichs eines Gehirns mit einer Aufnahmevorrichtung zur Erfassung der räumlichen Struktur des Gehirns, einer Rechenvorrichtung zur Erzeugung eines Simulationsmodells der Gehirnoberfläche, mindestens einer Stimulationsvorrichtung, insbesondere einer Spule, wobei mit einer Navigationsvorrichtung die Stimulationsvorrichtung unter Verwendung von Markern, welche am Kopf und/oder am Gehirn und/oder an der Stimulationsvorrichtung zum Navigieren und Positionieren der Stimulationsvorrichtung relativ zum Gehirn vorgesehen sind, so relativ zur Gehirnoberfläche positioniert werden kann, dass das von der Stimulationsvorrichtung erzeugbare magnetische Feld senkrecht steht auf dem Simulationsmodell der Gehirnoberfläche oder dass das von der Stimulationsvorrichtung erzeugte magnetische Feld senkrecht steht auf der Gehirnoberfläche an oder über dem zu stimulierenden Bereich oder einen vorgegebenen Raumwinkel dazu aufweist und wobei die Vorrichtung so ausgebildet ist, dass ein Stimulationssignal nur freigegeben oder erzeugt wird, wenn die Abweichung der errechneten Position der Stimulationsvorrichtung von der tatsächlichen Position der Stimulationsvorrichtung kleiner als ein vorgegebener Wert ist oder einen vorgegebenen Winkelbereich einhält.

11. Vorrichtung nach Anspruch 10 mit einer Steuerung, mit welcher die Stimulationsvorrichtung positioniert werden kann, wie in einem Verfahren nach einem der Ansprüche 1 bis 8 beschrieben.

12. Vorrichtung nach Anspruch 10 oder 11, wobei eine Anzeigevorrichtung zur Anzeige der relativen Position von Kopf oder Gehirn zur Stimulationsvorrichtung und/oder zur Anzeige des durch die Stimulationsvorrichtung erzeugbaren oder erzeugten Stimulationssignals, insbesondere eines induzierten elektrischen Feldes oder Signals vorgesehen ist.

13. Verfahren zum Bestimmen der Funktion eines bestimmten Bereiches des Gehirns, wobei eine Stimulationsvorrichtung nach Anspruch 1 positioniert wird und mindestens ein bestimmter Bereich durch die Stimulationsvorrichtung stimuliert wird und an mindestens zwei verschiedenen Positionen eine Messung der Reizantwort vorgenommen wird.

14. Verfahren nach Anspruch 13, wobei mindestens zwei, bevorzugt mehr auf einer Gitterstruktur liegende Positionen zum sequentiellen oder parallelen Stimulieren verschiedener Bereiche des Gehirns vorgesehen sind.

15. Verfahren nach Anspruch 13 und 14, wobei die mindestens zwei Reizerfassungsvorrichtungen die Stärke der Reizantwort erfassen.

16. Verfahren nach einem der Ansprüche 13 bis 15, wobei Stimulationssignale im Bereich des motorischen Cortex angelegt werden.

17. Verfahren nach einem der Ansprüche 13 bis 16, wobei eine computergeführte oder navigierte Stimulationsvorrichtung zur Erzeugung von Stimulationssignalen verwendet wird.

18. Verfahren nach einem der Ansprüche 13 bis 16, wobei zum Positionieren der mindestens einen Stimulationsvorrichtung ein Verfahren nach einem der Ansprüche 1 bis 9 verwendet wird.

19. Verfahren nach einem der Ansprüche 13 bis 18, wobei eine Mittelwertbildung aus mehreren Reizantworten bei im Wesentlichen gleichen Stimulationssignalen durchgeführt wird.

20. Verfahren nach einem der Ansprüche 13 bis 19, wobei eine Reizempfindlichkeitsfunktion für mindestens einen Muskel in Abhängigkeit von der Position auf der Gehirnoberfläche erzeugt wird.

21. Computerprogramm, welches das Verfahren nach einem der Ansprüche 13 bis 20 ausführt, wenn es in einen Computer geladen ist oder auf einem Computer läuft.

22. Programmspeichermedium oder Computerprogrammprodukt mit dem Programm nach Anspruch 21.

23. Vorrichtung zum Bestimmen der Funktion eines bestimmten Bereichs des Gehirns mit einer Vorrichtung nach Anspruch 10 und mindestens zwei Reizerfassungsvorrichtungen.

24. Vorrichtung nach Anspruch 23, wobei mindestens zwei, bevorzugt 16 oder mehr Stimulationsvorrichtungen vorgesehen sind, welche in einer Gitterstruktur angeordnet sind.

25. Vorrichtung nach Anspruch 23 oder 24 mit einer Steuervorrichtung zur Durchführung eines Verfahrens nach einem der Ansprüche 1 bis 7 und/oder 13 bis 20.

## Claims

1. A method for positioning and controlling a stimulation device relative to a point or area of the brain, wherein:
a) the spatial structure of the brain is recorded;
b) a three-dimensional simulation model of the surface of the brain is generated from the recording of the spatial structure of the brain;
c) the stimulation device is arranged relative to the brain using the three-dimensional simulation model of the surface of the brain, such that the at least one point or area of the brain can be stimulated using the stimulation device, and such that the magnetic field which can be generated by the stimulation device is vertical on the simulation model of the surface of the brain, or the magnetic field generated by the stimulation device is vertical on the surface of the brain on or over the area to be stimulated or exhibits a predetermined spatial angle relative to it; and
d) a stimulation signal is only released or generated when the deviation of the calculated position of the stimulation device from the actual position of the stimulation device is smaller than a predetermined value or remains within a predetermined angular range.

2. The method according to claim 1, wherein the spatial structure of the brain is recorded using a magnetic resonance method, a computer tomography method, an x-ray method or an ultrasound method.

3. The method according to any one of the preceding claims, wherein the simulation model of the surface of the brain is formed by a plurality of polygons.

4. The method according to any one of the preceding claims, wherein one or more coils are used as the stimulation device.

5. The method according to any one of the preceding claims, wherein the stimulation device is navigated to the calculated position for the desired stimulation.

6. The method according to any one of the preceding claims, wherein the positional data for the stimulation device and/or the brain and/or the electrical field which can be induced on the surface of the brain are displayed.

7. The method according to any one of the preceding claims, wherein a simulation model of the induction device and/or the brain is used, in particular a multi-shell model.

8. A computer program which performs the method according to any one of the preceding claims when it is loaded onto a computer or is running on a computer.

9. A program storage medium or computer program product comprising the program according to claim 8.

10. A device for stimulating and/or inhibiting at least one point or area of a brain, comprising a recording device for detecting the spatial structure of the brain, a computational device for generating a simulation model of the surface of the brain, at least one stimulation device, in particular a coil, wherein using a navigation device, the stimulation device can be positioned relative to the surface of the brain using markers provided on the head and/or on the brain and/or on the stimulation device for navigating and positioning the stimulation device relative to the brain, such that the magnetic field which can be generated by the stimulation device is vertical on the simulation model of the surface of the brain, or such that the magnetic field generated by the stimulation device is vertical on the surface of the brain on or over the area to be stimulated or exhibits a predetermined spatial angle relative to it, and wherein the device is designed such that a stimulation signal is only released or generated when the deviation of the calculated position of the stimulation device from the actual position of the stimulation device is smaller than a predetermined value or remains within a predetermined angular range.

11. The device according to claim 10, comprising a control system with which the stimulation device can be positioned, as described in a method according to any one of claims 1 to 8.

12. The device according to claim 10 or 11, wherein a display device is provided for displaying the position of the head or brain relative to the stimulation device and/or for displaying the stimulation signal which can be generated or which is generated by the stimulation device, in particular an induced electrical field or signal.

13. A method for determining the function of a particular area of the brain, wherein a stimulation device according to claim 1 is positioned and at least one particular area is stimulated using the stimulation device and the stimulus response is measured at at least two different positions.

14. The method according to claim 13, wherein at least two, preferably more, positions on a grid structure are provided for stimulating various areas of the brain sequentially or in parallel.

15. The method according to claim 13 and 14, wherein the at least two stimulus detection devices detect the strength of the stimulus response.

16. The method according to any one of claims 13 to 15, wherein stimulation signals are applied in the area of the motor cortex.

17. The method according to any one of claims 13 to 16, wherein a computer-guided or navigated stimulation device is used to generate stimulation signals.

18. The method according to any one of claims 13 to 16, wherein a method according to any one of claims 1 to 7 is used to position the at least one stimulation device.

19. The method according to any one of claims 13 to 18, wherein a mean value is formed from a number of stimulus responses for substantially the same stimulation signals.

20. The method according to any one of claims 13 to 19, wherein a stimulus sensitivity function is generated for at least one muscle, in accordance with the position on the surface of the brain.

21. A computer program which performs the method according to any one of claims 13 to 20 when it is loaded onto a computer or is running on a computer.

22. A program storage medium or computer program product comprising the program according to claim 21.

23. A device for determining the function of a particular area of the brain, comprising a device according to claim 10 and at least two stimulus detection devices.

24. The device according to claim 23, wherein at least two, preferably sixteen or more, stimulation devices are provided which are arranged in a grid structure.

25. The device according to claim 23 or 24, comprising a control device for performing a method according to any one of claims 1 to 7 and/or 13 to 20.

## Revendications

1. Procédé pour le positionnement et le guidage d'un dispositif de stimulation par rapport à un point ou une région d'un cerveau, comportant les étapes consistant à :
a) relever la structure spatiale du cerveau,
b) produire un modèle de simulation tridimensionnel de la surface du cerveau à partir du relevé de la structure spatiale du cerveau,
c) agencer le dispositif de stimulation par rapport au cerveau en utilisant le modèle de simulation tridimensionnel de la surface du cerveau, de sorte que le au moins un point ou la au moins une région du cerveau peut être stimulé(e) grâce au dispositif de stimulation et le champ magnétique pouvant être produit par le dispositif de stimulation est vertical perpendiculairement au modèle de stimulation de la surface du cerveau ou le champ magnétique produit par le dispositif de stimulation est vertical perpendiculairement à la surface du cerveau au niveau ou au-dessus de la région à stimuler ou présente par rapport à celle-ci un angle solide spécifié, et
d) ne libérer ou ne produire un signal de stimulation que quand l'écart de la position calculée du dispositif de stimulation par rapport à la position effective du dispositif de stimulation est inférieur à une valeur spécifiée, ou est compris un secteur angulaire spécifié.

2. Procédé selon la revendication 1, dans lequel le relevé de la structure spatiale du cerveau est mis en oeuvre grâce à un procédé de résonance magnétique nucléaire, de tomographie informatisée, de radiographie ou par ultrasons.

3. Procédé selon l'une quelconque des revendications précédentes, dans lequel le modèle de simulation de la surface du cerveau est formé d'une pluralité de polygones.

4. Procédé selon l'une quelconque des revendications précédentes, dans lequel une ou plusieurs bobines sont utilisées en tant que dispositif de stimulation.

5. Procédé selon l'une quelconque des revendications précédentes, dans lequel le dispositif de stimulation est piloté jusqu'à la position calculée pour la stimulation souhaitée.

6. Procédé selon l'une quelconque des revendications précédentes, dans lequel les données de position du dispositif de stimulation et/ou du cerveau et/ou du champ magnétique pouvant être induit au niveau de la surface du cerveau sont visualisées.

7. Procédé selon l'une quelconque des revendications précédentes, dans lequel un modèle de simulation du dispositif d'induction et/ou du cerveau, en particulier un modèle à plusieurs couches, est utilisé.

8. Programme informatique, qui exécute le procédé selon l'une quelconque des revendications précédentes, lorsqu'il est chargé dans un ordinateur ou est en fonctionnement dans un ordinateur.

9. Mémoire de programme ou produit informatique comportant le programme selon la revendication 8.

10. Dispositif de stimulation et/ou d'inhibition d'au moins un point ou d'au moins une région d'un cerveau, comportant un dispositif de relevé pour l'enregistrement de la structure spatiale du cerveau, un dispositif de calcul pour la production d'un modèle de simulation de la surface du cerveau, au moins un dispositif de stimulation, en particulier une bobine, le dispositif de stimulation pouvant être positionné par rapport à la surface du cerveau grâce à un dispositif de pilotage, en utilisant des marquages prévus au niveau de la tête et/ou au niveau du cerveau et/ou au niveau du dispositif de stimulation pour le pilotage et le positionnement du dispositif de stimulation par rapport au cerveau, de sorte que le champ magnétique pouvant être produit par le dispositif de stimulation est vertical perpendiculairement au modèle de simulation de la surface du cerveau ou le champ magnétique produit par le dispositif de stimulation est vertical perpendiculairement à la surface du cerveau au niveau de ou au-dessus de la région à stimuler ou présente par rapport à celle-ci un angle solide spécifié, et le dispositif étant réalisé de sorte qu'un signal de stimulation n'est libéré ou produit que quand l'écart de la position calculée du dispositif de stimulation par rapport à la position effective du dispositif de stimulation est inférieur à une valeur spécifiée, ou est compris un secteur angulaire spécifié.

11. Dispositif selon la revendication 10, comportant une commande grâce à laquelle le dispositif de stimulation peut être positionné, comme cela est décrit dans un procédé selon l'une quelconque des revendications 1 à 8.

12. Dispositif selon la revendication 10 ou 11, dans lequel un dispositif de visualisation pour la visualisation de la position relative de la tête ou du cerveau par rapport au dispositif de stimulation et/ou pour la visualisation du signal de stimulation pouvant être produit ou produit par le dispositif de stimulation, en particulier un signal ou un champ électrique induit, est prévu.

13. Procédé pour déterminer la fonction d'une région déterminée du cerveau, dans lequel un dispositif de stimulation selon la revendication 1 est positionné et au moins une région déterminée est stimulée grâce au dispositif de stimulation et une mesure de la réponse d'excitation est effectuée au niveau d'au moins deux positions distinctes.

14. Procédé selon la revendication 13, dans lequel au moins deux, de manière préférée plus de deux positions situées sur une structure réticulaire sont prévues pour la stimulation séquentielle ou parallèle de régions distinctes du cerveau.

15. Procédé selon la revendication 13 et 14, dans lequel les au moins deux dispositifs d'enregistrement d'excitation enregistrent la force de la réponse d'excitation.

16. Procédé selon l'une quelconque des revendications 13 à 15, dans lequel les signaux de stimulation sont appliqués dans la région du cortex moteur.

17. Procédé selon l'une quelconque des revendications 13 à 16, dans lequel un dispositif de stimulation guidé par ordinateur ou piloté est utilisé pour la production de signaux de stimulation.

18. Procédé selon l'une quelconque des revendications 13 à 16, dans lequel un procédé selon l'une quelconque des revendications 1 à 9 est utilisé pour le positionnement du au moins un dispositif de stimulation.

19. Procédé selon l'une quelconque des revendications 13 à 18, dans lequel une constitution de moyenne à partir de plusieurs réponses d'excitation est mise en oeuvre dans le cas de signaux de stimulation essentiellement identiques.

20. Procédé selon l'une quelconque des revendications 13 à 19, dans lequel une fonction de sensibilité d'excitation est produite pour au moins un muscle en fonction de la position sur la surface du cerveau.

21. Programme informatique, qui exécute le procédé selon l'une quelconque des revendications 13 à 20, lorsqu'il est chargé dans un ordinateur ou est en fonctionnement dans un ordinateur.

22. Support d'enregistrement de programme ou produit informatique comportant le programme selon la revendication 21.

23. Dispositif de détermination de la fonction d'une région déterminée du cerveau comportant un dispositif selon la revendication 10 et au moins deux dispositifs d'enregistrement d'excitation.

24. Dispositif selon la revendication 23, dans lequel au moins deux, de manière préférée seize ou plus de seize dispositifs de stimulation sont prévus, qui sont disposés selon une structure réticulaire.

25. Dispositif selon la revendication 23 ou 24, comportant un dispositif de commande pour la mise en oeuvre d'un procédé selon l'une quelconque des revendications 1 à 7 et/ou 13 à 20.
